# FASCICULE DE BREVET EUROPEEN

(11) **EP 1 043 015 B1**
(45) Date de publication et mention de la délivrance du brevet: **23.02.2005**
(21) Numéro de dépôt: 00400933.8
(22) Date de dépôt: 05.04.2000
(51) Int. Cl.: A61K 7/46

(54) **Composition parfumante liquide homogène à base de silicones volatiles**
Homogene flüssige Duftstoff-Zusammensetzung auf Basis von flüchtigen Silikonen
Homogeneous liquid fragrance composition based on volatile silicones

(30) Priorité: 07.04.1999 FR 9904315
(43) Date de publication de la demande: 11.10.2000
(73) Titulaire: CASTER, 75008 Paris (FR)
(72) Inventeur: Rodelet, François, 92100 Boulogne (FR)
(74) Mandataire: Casalonga, Axel

(56) Documents cités:
- EP-A- 0 723 776
- WO-A-96/03962
- US-A- 5 013 763

## Description

La présente invention concerne une composition parfumante liquide homogène exempte d'alcool, dans laquelle les molécules odorantes sont dissoutes dans une ou plusieurs silicones volatiles grâce à la présence de cosolvants appropriés.

Les produits de parfumerie tels que parfums, eaux de toilette, eaux de parfums etc., sont classiquement constitués d'une base parfumante, mélange complexe de molécules odorantes d'origine naturelle ou synthétique, en solution dans de l'éthanol ou dans un mélange hydroalcoolique à faible teneur en eau.

Ces produits à base d'alcool peuvent cependant provoquer chez certains utilisateurs des réactions indésirables telles qu'une mauvaise tolérance cutanée ou un dessèchement de la peau. Ils présentent par ailleurs un danger en cas d'absorption accidentelle par des enfants et font l'objet d'une surveillance particulière, notamment aux Etats Unis, suite à la sensibilisation récente de l'opinion publique aux problèmes écologiques résultant de l'émission de produits organiques volatils dans l'atmosphère.

C'est pourquoi des tentatives ont été faites pour solubiliser des bases parfumantes dans de l'eau à l'aide d'agents tensioactifs appropriés tels que l'huile de ricin polyéthoxylée. Les compositions ainsi obtenues ont été commercialisées sous forme d'eaux de toilette destinées essentiellement aux enfants.

Les quantités d'agents tensioactifs nécessaires sont importantes et les produits parfumants ainsi obtenus sont certes limpides mais fortement moussants et très collants sur la peau.

Une autre approche a consisté à dissoudre la base parfumante dans une huile ou un mélange d'huiles végétales. L'inconvénient de cette formule est d'être très grasse, laissant sur la peau un film désagréable pouvant tacher les vêtements.

Le but de la présente invention a été de mettre au point une composition parfumante liquide homogène exempte d'alcool présentant des propriétés cosmétiques et organoleptiques satisfaisantes (absence de toucher gras, de dessèchement ou d'irritation cutanée, aspect agréable, produit non moussant, etc.) et ayant une volatilité comparable à celle des compositions existantes à base d'alcool.

Cet objectif a été réalisé grâce à l'utilisation d'une ou de plusieurs silicones volatiles, linéaires ou cycliques, en association avec un ou plusieurs cosolvants particuliers appropriés, en tant que support liquide volatil capable de dissoudre les bases parfumantes connues utilisées habituellement en parfumerie.

La présente invention a donc pour objet une composition parfumante liquide homogène exempte d'alcool, constituée d'une base parfumante dissoute dans un support liquide volatil comprenant au moins une silicone volatile et au moins un premier cosolvant choisi parmi les esters d'alcools en C₄₋₁₅ et d'acides carboxylique en C₄₋₁₀, et, éventuellement, au moins un deuxième cosolvant choisi parmi certains diesters de polyéthylèneglycol oligomère.

Elle a en outre pour objet un procédé de préparation d'une telle composition parfumante liquide homogène exempte d'alcool consistant à dissoudre une base parfumante dans un support volatil comprenant au moins une silicone volatile, en utilisant comme agent de solubilisation
- au moins un premier cosolvant de type ester tel que défini ci-après et, éventuellement,
- au moins un deuxième cosolvant de type diester d'éthylèneglycol oligomère tel que défini ci-après.

L'invention a également pour objet l'utilisation d'au moins un premier cosolvant de type ester tel que défini ci-après, ou de l'association d'au moins un premier cosolvant de type ester et d'au moins un deuxième cosolvant de type diester d'éthlèneglycol oligomère tels que définis ci-après, pour solubiliser une base parfumante dans un support liquide à base de silicones volatiles.

Un autre objet de l'invention est l'utilisation en parfumerie d'une composition parfumante liquide homogène exempte d'alcool telle que décrite ci-dessus.

On entend par silicone volatile dans la présente invention des silicones ayant une pression de vapeur, mesurée à 25 °C, au moins égale à 5 pascals, de préférence au moins égale à 10 pascals.

Les silicones volatiles utilisables comme support liquide volatil de produits de parfumerie selon la présente invention sont choisies de préférence parmi les polydiméthylsiloxanes linéaires correspondant à la formule 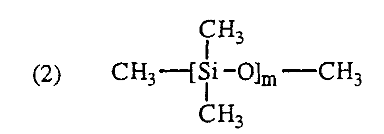 dans laquelle m est égal à 2, 3, 4 ou 5,
et les polydiméthylsiloxanes cycliques correspondant à la formule 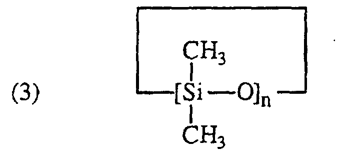 dans laquelle n vaut 4, 5 ou 6.

Au-delà du degré de polymérisation n ou m indiqué ci-dessus, c'est-à-dire au-delà de 5 motifs -Si(CH₃)-O- pour les siloxanes linéaires et 6 motifs -Si(CH₃)-O- pour les siloxanes cycliques, les silicones sont généralement trop peu volatiles pour pouvoir être utilisées dans la présente invention.

Ces silicones peuvent être utilisées seules ou sous forme de mélanges. Elles sont parfaitement miscibles entre elles en toutes proportions. Il est ainsi possible d'ajuster la vitesse d'évaporation d'un support liquide pour parfums en associant par exemple une silicone peu volatile à degré de polymérisation élevé à une silicone très volatile de faible masse.

Dans le groupe de silicones ci-dessus, on préfère en particulier l'octaméthyltrisiloxane linéaire (L₃), le décaméthyltétrasiloxane linéaire (L₄), l'octaméthyltétrasiloxane cyclique (D₄) et le décaméthylpentasiloxane cyclique (D₅).

Ces silicones ou mélanges de silicones sont généralement trop hydrophobes pour pouvoir dissoudre les bases parfumantes utilisées en parfumerie. Il a donc été nécessaire de trouver des agents de solubilisation appropriés, appelés ci-après cosolvants, c'est-à-dire des composés qui, lorsqu'ils sont utilisés en faible proportion par rapport aux silicones, permettent d'obtenir une solution homogène parfum/silicone.

Ces cosolvants doivent non seulement avoir un bon pouvoir solubilisant des molécules odorantes mais doivent également être non toxiques et posséder des propriétés cosmétiques satisfaisantes (absence de résidus gras, luisants ou collants).

Après de nombreux essais de solubilisation, la demanderesse a pu sélectionner un groupe de solvants organiques de type esters qui, seuls ou en combinaison, permettent de remplir les critères énoncés ci-dessus.

Ainsi, lorsque la base parfumante est relativement apolaire, on utilise, comme cosolvant, un ou plusieurs esters d'alcools en C₄₋₁₅ et d'acides carboxyliques en C₄₋₁₀.

Les alcools sont des alcools primaires aliphatiques, à chaîne linéaire ou ramifiée comportant de 4 à 15 atomes de carbone. On peut citer à titre d'exemple le butanol, le pentanol, l'hexanol, l'heptanol, l'octanol, l'alcool décylique, l'alcool dodécylique, l'alcool tétradécylique ou l'alcool pentadécylique.

Les acides sont des acides carboxyliques aliphatiques, cycloaliphatiques ou aromatiques, comportant de 4 à 10 atomes de carbone, parmi lesquels on préfère tout particulièrement l'acide benzoïque.

Les cosolvants particulièrement intéressants de ce premier type sont les benzoates d'alkyle à chaîne linéaire ou ramifiée en C₁₂₋₁₅, et on préfère tout particulièrement le benzoate de dodécyle.

D'autre part, lorsque la base parfumante présente une polarité importante, il peut être nécessaire d'associer à ce premier type de cosolvant, un deuxième type de cosolvant choisi parmi les diesters de polyéthylèneglycol de formule 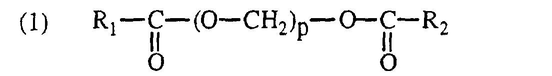 dans laquelle R₁ et R₂, identiques ou différents, représentent chacun un groupe alkyle en C₆₋₁₂ et p est égal à 3, 4 ou 5.

Un diester particulièrement préféré en tant que deuxième cosolvant est le diheptanoate de tétraéthylèneglycol.

La teneur en cosolvant(s) de la composition finale dépendra bien entendu de la nature chimique et de la quantité de molécules odorantes à solubiliser, de la ou des silicone(s) choisie(s) comme support volatil, de la nature chimique du ou des cosolvants et du rapport pondéral du premier au deuxième cosolvant éventuellement présent.

De manière générale, la composition parfumante finale contient de 0,5 à 10 parties en poids de cosolvant(s) pour 1 partie en poids de base parfumante.

La fraction de cosolvant ne dépasse généralement pas 25 % en poids de la composition finale.

Le rapport en poids du premier cosolvant au deuxième cosolvant, lorsque celui-ci est présent, est compris entre 1/1 et 100/1, de préférence entre 2/1 et 30/1.

Les bases parfumantes qui peuvent ainsi être solubilisées dans un support silicone volatile/cosolvant(s) sont choisies parmi les bases parfumantes utilisées habituellement en parfumerie. Il s'agit de mélanges complexes élaborés par les parfumeurs, composés d'huiles essentielles naturelles (extraites de fleurs, de plantes ou de certains organes animaux) associées ou non à des molécules odorantes obtenues par synthèse.

La composition parfumante de la présente invention peut être utilisée en tant qu'eau de toilette, eau de parfum, parfum ou lotion après rasage.

La présente invention sera décrite plus en détail à l'aide des exemples suivants qui ont un caractère purement illustratif.

### Exemple 1

| | |
|---|---|
| base parfumante composée principalement d'essence de Gardénia, de Jasmin et de Rose | 10 % en poids |
| benzoate de dodécyle | 10 % en poids |
| diheptanoate de tétraéthylèneglycol | 8 % en poids |
| hexaméthyldisiloxane | q.s.p. 100 % en poids |

### Exemple 2

| | |
|---|---|
| base parfumante composée principalement d'essences d'Iris, de Vanille et de Santal | 8 % en poids |
| benzoate de dodécyle | 3 % en poids |
| diheptanoate de tétraéthylèneglycol | 1 % en poids |
| octaméthyltétracyclosiloxane | q.s.p. 100 % en poids |

### Exemple 3

| | |
|---|---|
| base parfumante composée principalement d'essences de Mimosa, de Vanille et de Musc | 12 % en poids |
| benzoate de dodécyle | 8 % en poids |
| diheptanoate de tétraéthylèneglycol | 2 % en poids |
| octaméthyltrisiloxane | 45 % en poids |
| octaméthyltétracyclosiloxane | q.s.p. 100 % en poids |

### Exemple 4

| | |
|---|---|
| base parfumante composée principalement d'essences de Néroli, de Tubéreuse et de Vétyver | 6 % en poids |
| benzoate de dodécyle | 8 % en poids |
| diheptanoate de tétraéthylèneglycol | 4 % en poids |
| décaméthylpentacyclosiloxane | 10 % en poids |
| hexaméthyldisiloxane | q.s.p. 100 % en poids |

### Exemple 5

| | |
|---|---|
| base parfumante composée principalement d'essences de Lavande, de Vanille et de Bergamote | 6 % en poids |
| benzoate de dodécyle | 12 % en poids |
| diheptanoate de tétraéthylèneglycol | 4 % en poids |
| octaméthyltétracyclosiloxane | 68 % en poids |
| décaméthylpentacyclosiloxane | 8 % en poids |
| dodécaméthylhexacyclosiloxane | 2 % en poids |

### Exemple 6

| | |
|---|---|
| huile essentielle de lavande | 6 % en poids |
| benzoate de dodécyle | 10 % en poids |
| diheptanoate de tétraéthylèneglycol | 0,1 % en poids |
| octaméthyltrisiloxane | q.s.p. 100 % en poids |

## Revendications

1. Composition parfumante liquide homogène exempte d'alcool, constituée d'une base parfumante dissoute dans un support liquide volatil, **caractérisé par le fait que** ledit support liquide volatil comprend
- au moins une silicone volatile et
- au moins un premier cosolvant choisi parmi les esters d'alcools en C₄₋₁₅ et d'acides carboxyliques en C₄₋₁₀, et, éventuellement,
- au moins un deuxième cosolvant choisi parmi les diesters de formule 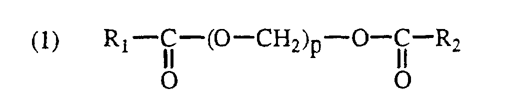
dans laquelle R₁ et R₂, identiques ou différents, représentent un groupe alkyle en C₆₋₁₂ et p est égal à 3, 4 ou 5.

2. Composition parfumante selon la revendication 1, **caractérisée par le fait que** la ou les silicones volatiles sont choisies parmi les polydiméthylsiloxanes linéaires correspondant à la formule 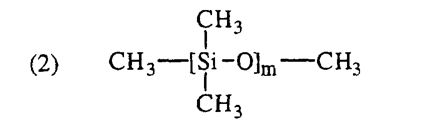 dans laquelle m est égal à 2, 3, 4 ou 5,
et les polydiméthylsiloxanes cycliques correspondant à la formule 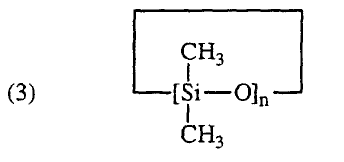 dans laquelle n vaut 4, 5 ou 6.

3. Composition parfumante selon la revendication 1 ou 2, **caractérisée par le fait que** la ou les silicones volatiles sont choisies parmi l'octaméthyltrisiloxane linéaire (L₃), le décaméthyltétrasiloxane linéaire (L₄), l'octaméthyltétrasiloxane cyclique (D₄) et le décaméthylpentasiloxane cyclique (D₅).

4. Composition parfumante selon l'une quelconque des revendications précédentes, **caractérisée par le fait que** ledit premier cosolvant est un benzoate d'alkyle à chaîne linéaire ou ramifiée en C₁₂₋₁₅.

5. Composition parfumante selon la revendication 4, **caractérisée par le fait que** ledit premier cosolvant est le benzoate de dodécyle.

6. Composition parfumante selon l'une quelconque des revendications précédentes, **caractérisée par le fait que** ledit deuxième cosolvant est le diheptanoate de tétraéthylèneglycol.

7. Composition parfumante selon l'une quelconque des revendications précédentes, **caractérisée par le fait qu'**elle contient de 0,5 à 10 parties en poids de cosolvant(s) pour 1 partie en poids de base parfumante.

8. Composition parfumante selon l'une quelconque des revendications précédentes, **caractérisée par le fait que** la fraction de cosolvant est inférieure à 25 % en poids par rapport à la composition finale.

9. Composition parfumante selon l'une quelconque des revendications précédentes, **caractérisée par le fait que** le rapport en poids du premier cosolvant au deuxième cosolvant, lorsque celui-ci est présent, est compris entre 1/1 et 100/1, de préférence entre 2/1 et 30/1.

10. Composition parfumante selon l'une quelconque des revendications précédentes, **caractérisée par le fait qu'**elle se présente sous forme d'un parfum, d'une eau de parfum, d'une eau de toilette ou d'une lotion après-rasage.

11. Procédé de préparation d'une composition parfumante liquide homogène exempte d'alcool, **caractérisé par le fait que** l'on dissout une base parfumante dans un support volatil comprenant au moins une silicone volatile, en utilisant comme agent de solubilisation
- au moins un premier cosolvant choisi parmi les esters d'alcools en C₄₋₁₅ et d'acides carboxyliques en C₄₋₁₀, et, éventuellement,
- au moins un deuxième cosolvant choisi parmi les diesters de formule 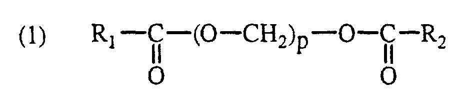
dans laquelle R₁ et R₂, identiques ou différents, représentent un groupe alkyle en C₆₋₁₂ et p est égal à 3, 4 ou 5.

12. Utilisation d'au moins un ester d'alcool en C₄₋₁₅ et d'acide carboxylique en C_{4-10'} en tant qu'agent solubilisant pour solubiliser une base parfumante dans un support liquide à base de silicones volatiles.

13. Utilisation de l'association d'au moins un ester d'alcool en C₄₋₁₅ et d'acide carboxylique en C₄₋₁₀, et d'au moins un diester de formule 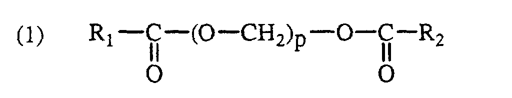 dans laquelle R₁ et R₂, identiques ou différents, représentent un groupe alkyle en C₆₋₁₂ et p est égal à 3, 4 ou 5,
en tant qu'agents solubilisants pour solubiliser une base parfumante dans un support liquide à base de silicones volatiles.

14. Utilisation en parfumerie d'une composition telle que définie dans l'une quelconque des revendications 1 à 10.

## Patentansprüche

1. Homogene flüssige und alkoholfreie Parfumzusammensetzung, die einen in einer flüchtigen Trägerflüssigkeit aufgelösten Basisriechstoff enthält, **dadurch gekennzeichnet, dass** die flüchtige Trägerflüssigkeit enthält:
- wenigstens ein flüchtigen Silikon und
- wenigstens ein erstes Hilfslösungsmittel, das möglicherweise unter den Estern von Alkoholen des Typs C₄₋₁₅ und den Carbonsäuren des Typs C₄₋₁₀ ausgewählt ist,
- wenigstens ein zweites Hilfslösungsmittel, das aus Diestern der Formel 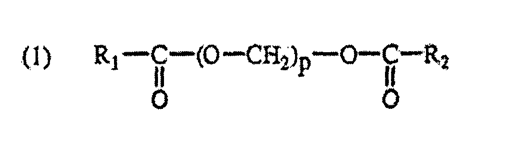
ausgewählt ist, wobei R₁ und R₂, ob identisch oder verschieden, eine Alkylgruppe des Typs C₆₋₁₂ repräsentieren, und p gleich 3, 4 oder 5 ist.

2. Parfumzusammensetzung nach Anspruch 1, **dadurch gekennzeichnet, dass** das oder die flüchtigen Silikone unter den linearen Polydimethylsiloxanen, die der Formel 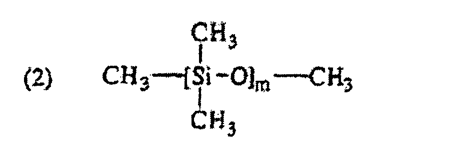 entsprechen, wobei m gleich 2, 3, 4 oder 5 ist, oder den zyklischen Polydimethylsiloxanen ausgewählt sind, die der Formel 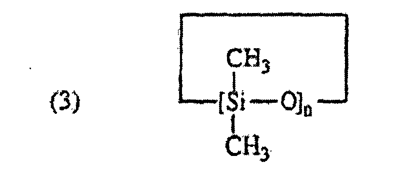 entsprechen, mit n gleich 4, 5 oder 6.

3. Parfumzusammensetzung nach Anspruch 1 oder 2, **dadurch gekennzeichnet, dass** das oder die flüchtigen Silikone unter dem linearen Octamethyltrisiloxan (L₃), dem linearen Decamethyltetrasiloxan (L₄), dem zyklischen Octamethyltetrasiloxan (D₄) und dem zyklischen Decamethylpentasiloxan (D₅) ausgewählt sind.

4. Parfumzusammensetzung nach einem der vorhergehenden Ansprüche, **dadurch gekennzeichnet, dass** das erste Hilfslösungsmittel ein Benzoat eines Alkyls mit einer linearen oder verzweigten Kette des Typs C₁₂₋₁₅ ist.

5. Parfumzusammensetzung nach Anspruch 4, **dadurch gekennzeichnet, dass** das erste Hilfslösungsmittel das Benzoat von Dodecyl ist.

6. Parfumzusammensetzung nach einem der vorhergehenden Ansprüche, **dadurch gekennzeichnet, dass** das zweite Hilfslösungsmittel das Deheptanoat von Tetraethylenglycol ist.

7. Parfumzusammensetzung nach einem der vorhergehenden Ansprüche, **dadurch gekennzeichnet, dass** diese zwischen 0,5 bis 10 Gewichtsteile an Hilfslösungsmittel(n) für 1 Gewichtsteil Basisriechstoff enthält.

8. Parfumzusammensetzung nach einem der vorhergehenden Ansprüche, **dadurch gekennzeichnet, dass** der Anteil an Hilfslösungsmittel bezogen auf die endgültige Zusammensetzung geringer als 25 % Gewichtsprozent ist.

9. Parfumzusammensetzung nach einem der vorhergehenden Ansprüche, **dadurch gekennzeichnet, dass** das Gewichtsverhältnis des ersten Hilfslösungsmittels gegenüber dem zweiten Hilfslösungsmittel, wenn dieses vorhanden ist, im Bereich von 1:1 bis 100:1, vorzugsweise im Bereich von 2:1 und 30:1 liegt.

10. Parfumzusammensetzung nach einem der vorhergehenden Ansprüche, **dadurch gekennzeichnet, dass** diese in Form eines Parfums, eines Eau de Parfum, eines Eau de Toilette oder einer After-shave-Lotion vorliegt.

11. Verfahren zum Herstellen einer homogenen, flüssigen und alkoholfreien Parfumzusammensetzung, **dadurch gekennzeichnet, dass** ein Basisriechstoff in einem flüchtigen Trägerstoff aufgelöst wird, der wenigstens ein flüchtiges Silikon enthält, wobei als eine die Löslichkeit fördernde Substanz verwendet wird:
- wenigstens ein erstes Hilfslösungsmittel, das unter den Estern der Alkohole des Typs C₄₋₁₅ und den Carbonsäuren des Typs C₄₋₁₀ ausgewählt wird, und möglicherweise
- wenigstens ein zweites Hilfslösungsmittel, das unter den Diestern der Formel 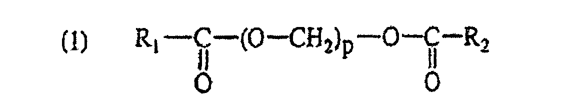
ausgewählt wird, wobei R₁ und R₂, ob identisch oder verschieden, eine Alkylgruppe des Typs C₆₋₁₂ repräsentieren, und p gleich 3, 4 oder 5 ist.

12. Verwenden wenigstens eines Alkoholesters des Typs C₄₋₁₅ und der Carbonsäure des Typs C₄₋₁₀, sowie Lösungsmittel, um einen Basisriechstoff in einer Trägerflüssigkeit basierend auf volatilen Silikonen löslich zu machen.

13. Verwenden der Verbindung wenigstens eines Alkoholesters des Typs C₄₋₁₅ und der Carbonsäure des Typs C₄₋₁₀, und wenigstens eines Diesters der Formel 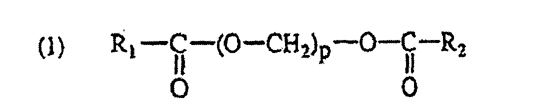 wobei R₁ und R₂, ob identisch oder verschieden, eine Alkylgruppe des Typs C₆₋₁₂ repräsentieren, und p gleich 3, 4 oder 5 ist, sowie Lösungsmittel, um einen Basisriechstoff in einer Trägerflüssigkeit basierend auf flüchtigen Silikonen löslich zu machen.

14. Verwenden einer Zusammensetzung, wie sie in einem beliebigen der Ansprüche 1 bis 10 definiert ist, in der Kosmetikindustrie.

## Claims

1. Alcohol-free, homogeneous liquid fragrancing composition consisting of a fragrancing base dissolved in a volatile liquid support, **characterized in that** the said volatile liquid support comprises
- at least one volatile silicone and
- at least one first co-solvent chosen from esters of C₄₋₁₅ alcohols and of C₄₋₁₀ carboxylic acids, and, optionally,
- at least one second co-solvent chosen from the diesters of formula 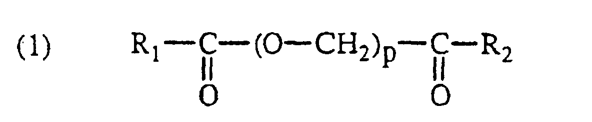
in which R₁ and R₂, which may be identical or different, represent a C₆₋₁₂ alkyl group and p is equal to 3, 4 or 5.

2. Fragrancing composition according to Claim 1, **characterized in that** the volatile silicone(s) is(are) chosen from linear polydimethylsiloxanes corresponding to the formula 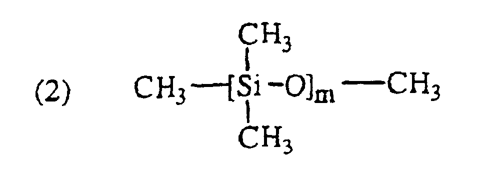 in which m is equal to 2, 3, 4 or 5,
and the cyclic polydimethylsiloxanes corresponding to the formula 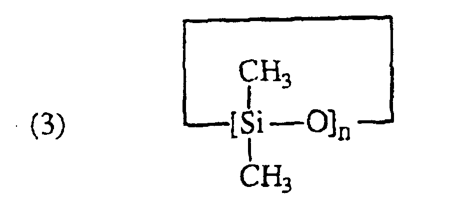 in which n is 4, 5 or 6.

3. Fragrancing composition according to Claim 1 or 2, **characterized in that** the volatile silicone(s) is (are) chosen from linear octamethyltrisiloxane (L₃), linear decamethyltetrasiloxane (L₄), cyclic octamethyltetrasiloxane (D₄) and cyclic decamethylpentasiloxane (D₅).

4. Fragrancing composition according to any one of the preceding claims, **characterized in that** the said first co-solvent is an alkyl benzoate containing a linear or branched C₁₂₋₁₅ chain.

5. Fragrancing composition according to Claim 4, **characterized in that** the said first co-solvent is dodecyl benzoate.

6. Fragrancing composition according to any one of the preceding claims, **characterized in that** the said second co-solvent is tetraethylene glycol diheptanoate.

7. Fragrancing composition according to any one of the preceding claims, **characterized in that** it contains from 0.5 to 10 parts by weight of co-solvent(s) per 1 part by weight of fragrancing base.

8. Fragrancing composition according to any one of the preceding claims, **characterized in that** the fraction of co-solvent is less than 25% by weight relative to the final composition.

9. Fragrancing composition according to any one of the preceding claims, **characterized in that** the weight ratio of the first co-solvent to the second co-solvent, when the latter is present, is between 1/1 and 100/1, preferably between 2/1 and 30/1.

10. Fragrancing composition according to any one of the preceding claims, **characterized in that** it is in the form of a perfume, an eau de parfum, an eau de toilette or an aftershave lotion.

11. Process for preparing an alcohol-free, homogeneous liquid fragrancing composition, **characterized in that** a fragrancing base is dissolved in a volatile support comprising at least one volatile silicone, using, as solubilizing agent
- at least one first co-solvent chosen from esters of C₄₋₁₅ alcohols and of C₄₋₁₀ carboxylic acids, and, optionally,
- at least one second co-solvent chosen from the diesters of formula 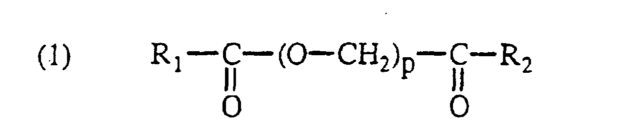
in which R₁ and R₂, which may be identical or different, represent a C₆₋₁₂ alkyl group and p is equal to 3, 4 or 5.

12. Use of at least one ester of a C₄₋₁₅ alcohol and of a C₄₋₁₀ carboxylic acid, as a solubilizing agent for dissolving a fragrancing base in a liquid support based on volatile silicones.

13. Use of a combination of at least one ester of a C₄₋₁₅ alcohol and of a C₄₋₁₀ carboxylic acid, and of at least one diester of formula 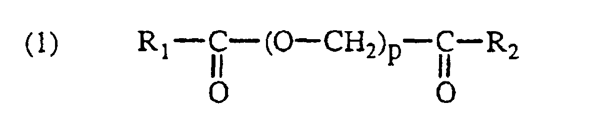 in which R₁ and R₂, which may be identical or different, represent a C₆₋₁₂ alkyl group and p is equal to 3, 4 or 5 as solubilizing agents for dissolving a fragrancing base in a liquid support based on volatile silicones.

14. Use in perfumery of a composition as defined in any one of Claims 1 to 10.
